# EUROPEAN PATENT SPECIFICATION

(11) **EP 0 482 076 B1**
(45) Date of publication and mention of the grant of the patent: **26.04.1995**
(21) Application number: 90911344.1
(22) Date of filing: 16.07.1990
(51) Int. Cl.: A61K 39/39, A61K 39/00

(54) **STABLE VACCINE COMPOSITIONS CONTAINING INTERLEUKINS**
STABILE INTERLEUKINE ENTHALTENDE IMPFSTOFFZUSAMMENSETZUNGEN
COMPOSITIONS DE VACCIN STABLE CONTENANT DES INTERLEUKINES

(30) Priority: 14.07.1989 US 379742
(43) Date of publication of application: 29.04.1992
(73) Proprietor: AMERICAN CYANAMID COMPANY, Portland, Maine 04101 (US)
(72) Inventor: PILLAI, Subramonia, Rochester, NY 14623 (US); BIXLER, Garvin, Knoxville, MD 21758 (US)
(74) Representative: Allam, Peter Clerk
(86) International application number: US9003982
(87) International publication number: WO9101143

(56) References cited:
- EP-A- 0 343 480
- EP-A- 0 351 876
- GB-A- 2 217 600
- JOURNAL OF IMMUNOLOGY, vol. 140, no. 1, 01 January 1988, American Assocation of Immunologists, Baltimore, MD (US); A. WEINBERG et al., pp. 294-299#
- ROTE LISTE 1989, Bundesverband der Pharmazeutischen Industrie e.V., Editio Cantor, Aulendorf/Würtemberg (DE); no. 74060#
- JOURNAL OF IMMUNOLOGY, vol. 141, no. 3, 01 August 1988, American Association of Immunologists, Baltimore, MD (US); M.F. GOOD et al., pp. 972-977#
- JOURNAL OF IMMUNOLOGY, vol. 139, no. 3, 01 August 1987, American Association of Immunologists, Baltimore, MD (US); L. NENCIONI et al., pp. 800-804#

## Description

### Background

It is often desirable to enhance the immunogenic potency of an antigen in order to obtain a stronger immune response in the organism being immunized and to strengthen host resistance to the antigen-bearing agent. A substance that enhances the immunogenicity of an antigen with which it is injected is called an adjuvant. One of the more effective adjuvants is Freund's adjuvant, a water-in-oil emulsion. Freund's adjuvant is most effective when live or killed mycobacteria are suspended in the emulsion (Freund's complete adjuvant) along with antigen. However, the intense, chronic inflammation that results around deposits of the emulsion precludes the use of the adjuvant in man. Emulsions lacking mycobacteria (incomplete Freund's adjuvant) are less irritating and have been used in man. Another type of adjuvant is a suspension of minerals on which antigen is adsorbed.

Certain lymphokines have been shown to have adjuvant activity thereby enhancing the immune response to an antigen. For example, Good et al. demonstrate the use of recombinant human IL-2 (rhIL-2) adsorbed on alum to enhance the immune response to a malaria related antigen. This composition was prepared and used immediately and stability over time was not ascertained. Good, M. F. et al., J. Immunol. 141:972-977 (1988). Nakamura et al. demonstrated that interferon-gamma induced a two- to five-fold enhancement of antibody formation to several antigens. Nakamura et al., Nature 307: 381-382 (1984). Interleukins have also been shown to enhance an immune response to other antigens. Nencioni et al, J. Immunol 139:800-804 (1987); Howard et al, EP285441. Weinberg et al., J. Immunol. 140:294-299 (1988) describe the use of recombinant IL-2 as an adjuvant for herpes simplex virus subunit vaccines. Incidence of infection was lower in animals that received IL-2. EP-A-343,480 also describes using IL-2 together with an antigen in a vaccine composition in particular for preparation of a Hepatitis B vaccine. Kishimoto et al., (EP-A-O 351,876) describe compositions for potentiating vaccination effect using human B-cell differentiation factor as the effective ingredient. The composition can additionally contain one or more cytokines, such as interleukin and interferon.

### Summary of the Invention

This invention pertains to stable interleukin-containing vaccine compositions comprising a mixture of antigen and an adjuvant amount of an interleukin adsorbed onto alum (eg aluminum hydroxide or aluminum phosphate) in suspension. The mixture can comprise a preservative. Interleukins, such as interleukin-1α, interleukin-1β, interleukin-2, interleukin-3, interleukin-4, interleukin-5, interleukin-6 and interleukin-7 can be used as adjuvants in combination with antigen (particularly glycoconjugates) adsorbed onto a alum (eg aluminum hydroxide or phosphate) to modulate the immune response to the antigen. The vaccine compositions can be stored.

### Detailed Description of the Invention

The vaccine compositions of this invention comprise an adjuvant amount of an interleukin in combination with the antigen adsorbed onto a mineral in suspension and a preservative. Preferably, the mineral is alum (e.g., aluminum hydroxide or aluminum phosphate) which is suspended in an aqueous medium.

The interleukin functions to modulate the immune response to the antigen, while the alum stabilizes the biological activity of the interleukin. In the absence of alum, interleukins have short half lives. Thus, the vaccine compositions of the present invention can be stored for periods of time which would otherwise result in the destabilization of the interleukin. Stabilization will greatly extend the permissible time for the manufacture, shipment and storage of the vaccine formulations prior to administering of the vaccine formulation.

Several different interleukins can be used. These include interleukin-1α, interleukin-1β, interleukin-2, interleukin-3, interleukin-4, interleukin-5, interleukin-6, interleukin-7, or mixtures of these. Portions of interleukins having immunomodulating activity can also be used. The preferred interleukin is interleukin-2.

Interleukin can be obtained from any suitable source. They can be produced by recombinant DNA methodology. For example, the genes encoding several human interleukins have been cloned and expressed in a variety of host systems, permitting the production of large quantities of pure human interleukin. Further, certain T lymphocyte lines produce high levels of interleukin thus providing a source of the interleukin.

The preservative can be any pharmaceutically acceptable preservative. These include thimerosal, phenol, m-cresol, benzyl alcohol, methyl or ethyl paraben, and 2-phenoxyethanol.

Interleukin can be used as adjuvant for many different types of antigens. In general, the antigens can be particulate antigens such as bacteria, viruses and macrocomponents of cells and soluble antigens such as proteins, peptides, glycoproteins and carbohydrates. Antigens of particular interest are viral or bacterial antigens, allergens, auto-immunity related antigens, tumor-associated antigens, oncogene products, parasite antigens, fungal antigens or fragments of these. The antigens can be obtained from natural sources or they can be produced by recombinant DNA technology or other artificial means.

Among the bacterial antigens of interest are those associated with the human bacterial pathogens including, for example, typable and nontypable Haemophilus influenzae, Escherichia coli, Neisseria meningitidis, Streptococcus pneumoniae, Streptococcus pyogenes, Branhamella catarrhalis, Vibrio cholerae, Corynebacteria diphtheriae, Neisseria gonorrhoeae, Bordetella pertussis, Pseudomonas aeruginosa, Staphylococcus aureus, Streptococcus pyogenes, Klebsiella pneumoniae, and Clostridium tetani. Some specific bacterial antigens include bacterial surface and outer membrane proteins (e.g. from Haemophilus influenzae, Neisseria meningitidis, Neisseria gonorrhoeae or Branhamella catarrhalis) and bacterial surface proteins (e.g. the M protein from Streptococcus pyogenes).

Viral antigens from pathogenic viruses include but are not limited to, human immunodeficiency virus (types I and II), human T-cell leukemia virus (types I, II and III), respiratory syncytial virus, hepatitis A, hepatitis B, hepatitis C, non-A and non-B hepatitis virus, herpes simplex virus (types I and II), cytomegalovirus, influenza virus, parainfluenza virus, poliovirus, rotavirus, coronavirus, rubella virus, measles virus, varicella, Epstein Barr virus, adenovirus, papilloma virus and yellow fever virus.

Several specific viral antigens of these pathogenic viruses include the F protein (especially antigens containing the F peptide 283-315 described in WO89/02935 entitled "Respiratory Syncytial Virus: Vaccines and Diagnostic Assays" by Paradiso, P. et al.) and the N and G proteins of respiratory syncytial virus (RSV), VP4 (previously known as VP3), VP6 and VP7 polypeptides of rotavirus, envelope glycoproteins of human immunodeficiency virus, the S and pre-S antigens of hepatitis B and herpes glycoproteins B and D.

Also of interest are various antigens associated with auto-immune diseases, such as rheumatoid arthritis and lupus erythematosus.

Of particular interest for use in a vaccine are capsular polymers (CP) produced by bacterial pathogens. Capsular polymers are sugar containing polymers, such as polymers of sugars, sugar acids, amino sugars, polyhydric alcohols and sugar phosphates. Several capsular polymers and oligomers are useful as vaccines.

The capsular polymers (CP) can be derived from many different types of bacteria. These types include Haemophilus influenzae, Streptococcus species including pneumoniae (particularly serotypes 1, 4, 5, 6A, 6B, 9V, 14, 18C, 19F, and 23F) pyogenes and agalactiae, Neisseria meningitidis (such as serogroup a, b and c), Klebsiella pneumoniae, Pseudomonas aeruginosa and Staphylococcus aureus.

Non-bacterial polymers can be derived from yeast and fungi, for example, Cryptococcus neoformans, or carbohydrate containing units found uniquely on cancer cells or those found associated with allergens.

The antigens of this invention can be used to elicit an immune response to an antigen in a vertebrate (such as a mammalian host). The method comprises administering to the animal, an immunologically effective dose of a vaccine composition comprising a mixture of an antigen and an adjuvant amount of an interleukin adsorbed onto a mineral in suspension and an added preservative. The vaccine compositions are useful for the prevention of microbial infections. The antigens may be administered in a pharmaceutically acceptable vehicle, such as physiological saline, or ethanol polyols (such as glycerol or propylene glycol). The antigen may be coupled to a glycoconjugate selected from a bacterial toxin of diphtheria, tetanus, pertussis or cross reacting material (CRM), or toxoid thereof and in particular polyribosylribitolphosphate and CRM₁₉₇ of diphtheria toxin. The vaccine compositions may optionally comprise other adjuvants, such as vegetable oils or emulsions thereof, surface active substances, e.g., hexadecylamine, octadecyl amino acid esters, octadecylamine, lysolecithin, dimethyl-dioctadecylammonium bromide, N,N-dicoctadecyl-N'-N'bis (2-hydroxyethyl-propane diamine), methoxyhexadecylglycerol, and pluronic polyols; polyamines, e.g., pyran, dextransulfate, poly IC, carbopol; peptides, e.g., muramyl dipeptide, dimethylglycine, tuftsin; immune stimulating complexes (ISCOMS); oil emulsions; and mineral gels. The antigens of this invention may also be incorporated into liposomes or ISCOMS. Supplementary active ingredients may also be employed.

The vaccines can be administered to a human or animal in a variety of ways. These include intradermal, transdermal (such as by the use of slow release polymers), intramuscular, intraperitoneal, intravenous, subcutaneous, oral and intranasal routes of administration. The amount of antigen employed in such vaccine will vary depending upon the identity of the antigen employed. Adjustment and manipulation of established dosage ranges used with traditional carrier antigens for adaptation to the present vaccines is well within the ability of those skilled in the art. The vaccines of the present invention are intended for use in the treatment of both immature and adult warm-blooded animals, and in particular humans. Also, the use of the present composition is not limited to prophylactic application; therapeutic application are also contemplated (e.g., AIDS prophylaxis and therapy).

The adjuvant action of interleukin has a number of important implications: the adjuvant action of the interleukin can increase the concentration of protective antibodies produced against the antigen in the vaccinated organism. As a result, effective (i.e., protective) vaccination can be achieved with a smaller quantity of antigen than would be normally required. This reduction in the required amount of antigen may lead to more wide-spread use of vaccines which are difficult or costly to prepare. This is especially true in the developing nations which have very limited health care budgets and which face epidemics of respiratory diseases, diarrheal diseases and malaria. It may also provide for safer vaccination when the antigen is toxic at the concentration normally required for effective immunization. By reducing the amount of antigen, the risk of toxic reaction is reduced.

Interleukins, by means of their immunomodulating activity, can help evoke a protective immune response against marginally or non-immunogenic antigens. In this manner, vaccine compositions containing fragments of larger proteins, synthetic antigens or products of recombinant DNA technology may be made more potent by mixing them with interleukins.

Typically, vaccination regimens call for the administration of antigen over a period of weeks or months in order to stimulate a "protective" immune response. A protective immune response, is an immune response sufficient to protect the immunized host or subject from productive infection by a particular pathogen or pathogens to which the vaccine is directed. Interleukin, when coadministered with antigen and adsorbed onto a mineral in suspension can accelerate the generation of a protective immune response. This may reduce the time course of effective vaccination regimens. In some instances, it may result in the generation of a protective immune response in a single dosage. Further, vaccine formulations of this invention are sufficiently stable at 4°C to allow the manufacture, shipment and storage of the vaccine formulations.

This invention is further illustrated by the following examples.

### EXAMPLES

### Example I: Adjuvant Effect of rhIL-1 or rhIL-2 in the Presence of Alum With an HbOC Antigen Vaccine

A low dose (0.1 »g/mouse) dose of Haemophilus type b CRM conjugate (HbOC) was formulated with or without alum (100 »g/mouse) and with various concentrations of rhIL-2, rhIL-1α or rhIL-1β ranging from 1 x 10² - 5 x 10⁵ units/mouse. All vaccines were prepared on the day of immunization and maintained at 4°C until injected. Interleukins used in the preparation of the vaccines were either reconstituted on the day of formulation or were recovered from diluted stock (25 »g/ml) maintained at - 70°C. Groups of Swiss-Webster mice (Taconic Farms, Germantown, NY) were injected intramuscularly (I.M.) at weeks 0 and 2 with 0.1 ml of the vaccine formulations. Serum samples were collected as indicated in the various Tables.

### A. rhIL-2 as Adjuvant

Table 1 shows the results observed when rhIL-2 was administered as adjuvant in a mouse vaccination, both in the absence of alum [columns denoted (-)], and in combination with alum [columns denoted (+)]. Antibody concentrations are expressed as »g/ml and were determined at weeks 2, 4, and 6, following the administration of 0.1 »g of the antigen. The antigen used in these studies was HbOC. Doses of rhIL-2 administered ranged from 1 X 10³ - 1 X 10⁵ units/mouse.

**TABLE 1**

| HbOC »g | Units rhIL | Week 2 | | Week 4 | | Week 6 | |
|---|---|---|---|---|---|---|---|
| | | (+) | (-) | (+) | (-) | (+) | (-) |
| Series 1: | | | | | | | |
| 0.1 | 10³ | 4.70 | 0.28 | 7.89 | 3.46 | 8.16 | 3.62 |
| 0.1 | 10⁴ | 2.25 | 0.51 | 9.39 | 5.01 | 17.77 | 5.83 |
| 0.1 | 10⁵ | 0.37 | 0.55 | 4.71 | 3.04 | 5.31 | 2.91 |
| 0.1 | -- | 0.46 | <0.10 | 3.53 | 0.42 | 5.46 | 3.53 |

| Series 2: | | | | | | | |
|---|---|---|---|---|---|---|---|
| 0.1 | 10² | 1.02 | 0.40 | 6.32 | 4.21 | 5.90 | 3.32 |
| 0.1 | 10³ | 0.54 | 0.75 | 5.77 | 4.73 | 5.60 | 4.61 |
| 0.1 | 10⁴ | 1.68 | <0.10 | 12.99 | 1.95 | 19.06 | 1.25 |
| 0.1 | 10⁵ | 0.25 | <0.10 | 5.94 | 5.46 | 3.91 | 3.82 |
| 0.1 | -- | 0.53 | 0.30 | 7.09 | 2.64 | 8.30 | 2.82 |

As can be seen in the columns denoted (-) above, rhIL-2 without alum does have a stimulatory effect on antibody production For example, in two separate experiments, the antibody concentration present in the control samples was 0.42 »g/ml and 2.62 »g/ml.

The results observed when alum was included with rhIL-2 in the adjuvant mixture are shown in the columns denoted (+) in Table 1. Again, a significant stimulatory effect is seen. However, when alum is present, the magnitude of the antibody response is significantly increased over the response observed with otherwise identical samples without alum (vaccinations).

Again considering the 4 week time point, it can be seen that the with (+) alum antibody concentrations are up to more than 10-fold greater than the corresponding without (-) alum antibody concentrations. Furthermore, there is an apparent rhIL-2 concentration dependence which was absent in the samples without (-) alum. Specifically, the correlation observed was that antibody concentration increased, as the rhIL-2 concentration decreased below 1 x 10⁵. In the case of rhIL-2 with alum, the dose which appeared to stimulate the highest antibody production was approximately 1 x 10⁴ units per mouse.

### B. rhIL-1 as Adjuvant

Tables 2 and 3 show results obtained from immunizations in which rhIL-1 α and rhIL-1β were used as adjuvant, respectively. Table 2 presents data obtained from anti-PRP antibody determinations (expressed in »g/ml) at biweekly intervals post-immunization. Again, the experiments were conducted either with, (+), or without, (-), alum.

**TABLE 2**

| HbOC »g | rhIL-1α (Units) | Week 2 | | Week 4 | | Week 6 | |
|---|---|---|---|---|---|---|---|
| | | (+) | (-) | (+) | (-) | (+) | (-) |
| Series 1: | | | | | | | |
| 0.1 | 10³ | 1.96 | 1.02 | 14.63 | 4.94 | 13.25 | 4.93 |
| 0.1 | 10⁴ | 0.70 | 2.54 | 7.16 | 5.11 | 12.44 | 11.17 |
| 0.1 | 10⁵ | 0.33 | 1.49 | 4.69 | 7.02 | 6.14 | 8.73 |
| 0.1 | -IL Control | 0.46 | <0.10 | 3.53 | 0.42 | 5.46 | 3.53 |

| Series 2: | | | | | | | |
|---|---|---|---|---|---|---|---|
| 0.1 | 10² | <0.10 | 1.02 | 3.68 | 4.36 | 4.07 | 3.00 |
| 0.1 | 10³ | 1.06 | 1.17 | 12.31 | 5.54 | 8.74 | 3.08 |
| 0.1 | 10⁴ | 0.47 | 1.47 | 4.73 | 8.97 | 5.98 | 6.55 |
| 0.1 | 10⁵ | 0.34 | 1.00 | 8.55 | 16.74 | 7.77 | 16.29 |
| 0.1 | -IL Control | 0.53 | 0.30 | 7.09 | 2.64 | 8.30 | 2.82 |

The results observed from rhIL-1α and rhIL-1β adjuvant mixtures were similar, overall, to those observed in the rhIL-2 series. Table 2, for example, shows that when rhIL-1α is administered as adjuvant without alum there is a stimulatory effect when compared with an otherwise identical vaccine without the lymphokine. As shown in the columns denoted (-), a tendency toward decreasing antibody production was observed as the amount of rhIL-1α in the adjuvant mixture was decreased from 1 x 10⁵ to 1 x 10² units per mouse.

In the presence of alum, rhIL-1α also had a stimulating effect. Surprisingly, rhIL-1α demonstrated increasing ability to stimulate the immune response as its concentration was decreased. The optimal adjuvant amount of rhIL-1α, in the presence of alum, was approximately 1 x 10³. At such concentrations the antibody concentration was found to be 2-3 fold greater than the otherwise identical samples without (-) alum.

Similar results were observed when rhIL-1β was used as adjuvant, as shown in Table 3. The data in Table 3 summarizes the results of antibody concentration determinations made at weeks 2, 4, and 6, for varying concentrations of rhIL-1β as adjuvant, either with or without alum. In all but 6 of 21 experimental groups, the vaccine with (+) alum resulted in a higher antibody concentration than the vaccine without (-) alum.

**TABLE 3**

| HbOC »g | rhIL-1β | Week 2 | | Week 4 | | Week 6 | |
|---|---|---|---|---|---|---|---|
| | | (+) | (-) | (+) | (-) | (+) | (-) |
| Series 1: | | | | | | | |
| 0.1 | 10³ | 0.41 | 0.96 | 5.27 | 4.09 | 8.32 | 5.22 |
| 0.1 | 10⁴ | 0.90 | 0.62 | 4.13 | 3.39 | 8.15 | 6.39 |
| 0.1 | 10⁵ | 1.46 | 0.46 | 13.35 | 1.77 | 8.60 | 1.06 |
| 0.1 | -IL Control | 0.46 | <0.10 | 3.53 | 0.42 | 5.46 | 3.53 |

| Series 2: | | | | | | | |
|---|---|---|---|---|---|---|---|
| 0.1 | 10² | 0.85 | 0.16 | 8.76 | 0.04 | 6.66 | 1.91 |
| 0.1 | 10³ | 0.30 | 0.40 | 4.57 | 0.68 | 6.87 | 10.33 |
| 0.1 | 10⁴ | 1.08 | 0.36 | 5.75 | 5.15 | 5.17 | 4.47 |
| 0.1 | 10⁵ | 0.19 | 0.36 | 2.34 | 3.0 | 4.98 | 3.92 |
| 0.1 | -IL Control | 0.53 | 0.30 | 7.09 | 2.64 | 8.30 | 2.82 |

### Example II: Adjuvant Effect of rhIL-1α, rhIL-1β, rhIL-2, and Mixtures Thereof on an RSV F Protein Vaccine

To determine whether interleukins can be used to enhance the antibody response to a protein vaccine, various concentrations of F protein of respiratory syncytial virus (RSV) were formulated with alum (final 100 »g/mouse) and with or without rhIL-2 or rhIL-1α at 1 x 10⁴ or 1 x 10⁵ units/mouse. Groups of Swiss-Webster mice (5 animals per group) were immunized intramuscularly at weeks 0 and 2. Animals were bled as indicated in Table 4.

Three doses of RSV F protein (1, 0.1 and 0.01 »g/mouse) were administered. Of these only 0.01 »g was suboptimal under the conditions employed. Comparison of the response seen in those groups receiving vaccines containing various interleukins with the control group receiving 0.01 »g of protein in alum alone revealed no significant effect (4 fold difference in titer) of rhIL-2 or rhIL-1α on the antibody response to F protein. However, the responses in the rhIL-2 treated groups were higher than controls at doses of 1 x 10⁴ units/mouse which is similar to the results obtained in the HbOC studies. With IL-1α, both doses seem to show some improvement in antibody responses over controls Interestingly, mixtures of 1 x 10⁴ units/mouse of rhIL-2 and rhIL-1α did not show any indication of synergy but rather showed a slight decrease in the response relative to controls suggesting a possible antagonistic interaction of the interleukins.

**TABLE 4**

| Adjuvant Effect of rhIL-2 on RSV F protein vaccine | | | | | |
|---|---|---|---|---|---|
| »gF protein | rhIL | Units | Week 2 | Week 4 | Week 6 |
| 0.1 | -IL Control | -- | 141,657 | 2,341,756 | 2,454,512 |
| 0.1 | IL-2 | 10⁴ | 191,935 | 2,045,327 | 2,101,951 |
| 0.1 | IL-2 | 10⁵ | 309,797 | 2,275,311 | 2,311,326 |
| 0.01 | -IL Control | -- | 68,627 | 802,611 | 687,334 |
| 0.01 | IL-2 | 10⁴ | 145,467 | 1,580,552 | 1,699,135 |
| 0.01 | IL-2 | 10⁵ | 60,134 | 722,396 | 815,351 |
| 0.1 | -IL Control | -- | 141,657 | 2,341,756 | 2,454,512 |
| 0.1 | IL-1α | 10⁴ | 123,446 | 1,861,917 | 1,771,952 |
| 0.1 | IL-1α | 10⁵ | 79,386 | 1,185,475 | 1,214,008 |
| 0.01 | -IL Control | -- | 68,627 | 802,611 | 687,334 |
| 0.01 | IL-1α | 10⁴ | 54,081 | 1,003,110 | 1,094,459 |
| 0.01 | IL-1α | 10⁵ | 59,566 | 708,009 | 1,178,056 |
| 0.1 | -IL Control | -- | 141,657 | 2,341,756 | 2,451,512 |
| 0.1 | Mix | 10⁴ | 207,410 | 2,593,957 | 2,353,912 |
| 0.01 | -IL Control | -- | 68,627 | 802,611 | 687,334 |
| 0.01 | Mix | 10⁴ | 15,947 | 367,224 | 532,050 |
| 1.0 | -- | -- | 211,662 | 2,269,615 | 2,899,079 |

### Example III. Single Dose Vaccination

Table 1 shows the results of an experiment demonstrating the immune response to HbOC with rhIL-2 with and without alum. At a concentration of 1 x 10³ units rhIL-2, in the presence of alum, an HbOC based vaccine stimulated an antibody response of 4.7 »g/ml, after a single administration. Such an antibody concentration is above the threshold level generally accepted as necessary for protection.

### Example IV. Adjuvant Stability

### A. In Vitro Assay of rhIL-2 Stability

To study the stability of rhIL-2 in an alum containing composition, HbOC antigen (2.5 »g/mouse) was mixed with 10⁴ units of rhIL-2 and adsorbed onto aluminum phosphate and stored at 4°C. Table 5 presents results observed in an in vitro stability assay (counts per minute (CPM) ± standard deviation (SD)). In the Assay, 5 X 10³ CTLL-2 (cytotoxic T lymphocyte lysis) cells were cultured with various concentrations of rhIL-2 standard (Boehringer Mannheim(BM) and Cetus products) and HbOC vaccines. Cells were incubated in Roswell Park Memorial Institute (RPMI) media containing 10% fetal bovine serum (FBS) at 37° for 24 hours and pulsed with 1.0 »Ci/well [³H]-thymidine for 16 hours to estimate the amount of rhIL-2 activity. It was determined that approximately 2/3 of the rhIL-2 is adsorbed onto alum. As shown in Table 5, when tested for rhIL-2 activity at time points following aluminum phosphate absorption, it was determined that the lymphokine maintained its activity for up to two weeks.

**TABLE 5**

| In vitro assay of rhIL-2 stability (Incorporation of [3H]-thymidine at 1.0 U/ml IL-2 A ±CPM ± SD). | | | |
|---|---|---|---|
| Baseline: | Week 0 | Week 1 | Week 2 |
| Media (x cpm) | 303 ± 41 | 1,540 ± 67 | 135 ± 49 |

| Interleukin-2: | | | |
|---|---|---|---|
| BM product | 16,179 ± 491 | 10,056 ± 689 | 13,323 ± 1428 |
| Cetus product | 28,300 ± 2250 | 25,681 ± 135 | 25,992 ± 868 |

| HbOC vaccines: | | | |
|---|---|---|---|
| rhIL-2 | 24,188 ± 783 | 31,989 ± 2252 | 36,312 ± 3102 |
| Alum/rhIL-2 | 19,419 ± 1330 | 28,785 ± 1691 | 35,091 ± 690 |
| Alum/rhIL-2 rhIL-2+50»g | supt 4,460 ± 205 | 5,716 ± 211 | 13,460 ± 1231 |
| Alum | ND | ND | 35,627 ± 2503 |

### B. In Vivo Assay of rhIL-2 Stability

An in vivo assay of rhIL-2 stability was designed. Mice were immunized with an HbOC/Alum/IL-2 vaccine which had been stored as described above. Four groups of two DBA/2 mice were immunized with 10»g (protein) HbOC in complete freuds adjuvant (CFA), Alum, rhIL-2/Alum, or rhIL-2 on 3 consecutive weeks. Lymph nodes were removed one week after injection and single cell suspension was obtained. 3 x 10⁵ lymph node cells (LNC) were cultured with mitogens and various concentrations of concanavalin A (CA), lipopolysaccharide (LPS) diphtheria toxin (DT), cross reacting material (CRM), and tetanus toxin (TT). Cells were incubated in RPMI media containing 1.0% normal mouse serum (NMS) at 37°C for 3 days, pulsed with 1.0»Ci/well [3H]-thymidine for 16 hrs, and harvested for counting on LS counter. (Maximum Incorporated [3H]-thymidine as stimulation index (SI) ± standard deviation (SD)). A significant T cell response was observed in the HbOC-CFA group. In weeks 2 and 3, however, rhIL-2 induced an augmented T cell response. Furthermore even HbOC alone appears to protect rhIL-2 from degradation.

**TABLE 6**

| In Vivo Stability of IL-2 with HbOC Vaccine Maximum Incorporated [3H] - thymidine as SI ± SD. | | | | |
|---|---|---|---|---|
| In Vitro Challenge: | Week 1 HbOC Priming In: | | | |
| | CFA | Alum* | IL-2/Alum* | IL-2* |
| Media (x cpm) | 454 ± 22 | 190 ±19 | 386 ±35 | 125 ± 8 |
| CA 1.0»g/ml | 43.3± 3.9 | 89.7± 3.9 | 58.4± 5.1 | 136.9± 2.4 |
| LPS 50»g/ml | 129.4± 8.7 | 189.9± 7.7 | 69.0± 4.5 | 103.9± 6.6 |
| DT | 17.5± 1.2 | 4.4± 0.04 | 1.4± 0.3 | 1.8± 0.1 |
| CRM | 162.0± 14.9 | 9.8± 0.3 | 0.8± 0.1 | 0.8± 0.1 |
| TT | 0.8± 0.07 | 0.4± 0.03 | 0.1± 0.0 | 0.6± 0.05 |

| | Week 2 | | | |
|---|---|---|---|---|
| Media | 1,185 ±175 | 590 ±42 | 552 ±48 | 187 ±11 |
| CA 1.0»g/ml | 40.6± 3.4 | 57.5± 1.8 | 53.1± 0.9 | 165.1±11.5 |
| LPS 50»g/ml | 71.5± 1.2 | 89.9± 3.7 | 142.1± 8.7 | 203.5±17.8 |
| DT | 16.5± 0.4 | 1.1± 0.0 | 1.3± 0.2 | 2.9± 0.0 |
| CRM | 50.6± 0.5 | 1.3± 0.0 | 10.5± 0.3 | 1.6± 0.0 |
| TT | 0.6± 0.1 | 0.1± 0.0 | 0.1± 0.0 | 0.4± 0.0 |

| | Week 3 | | | |
|---|---|---|---|---|
| Media | 519 ± 59 | 416 ± 9 | 442 ±63 | 966 ±51 |
| CA 1.0»g/ml | 45.9± 0.3 | 41.0± 2.8 | 46.4± 0.9 | 27.5± 2.1 |
| LPS 50»g/ml | 129.2± 7.6 | 119.4± 5.3 | 126.5± 6.3 | 81.9± 3.1 |
| DT | 5.6± 0.8 | NA | 2.2± 0.2 | NA |
| CRM | 98.1± 8.1 | 17.9± 1.4 | 25.2± 1.6 | 31.4± 2.6 |
| TT | 0.4± 0.1 | 0.2± 0.0 | 0.51 0.0 | 0.1± 0.0 |

| | | | | |
|---|---|---|---|---|
| *These groups received 2.0% NMS instead of 1.0% | | | | |

## Claims

1. A stable vaccine composition, comprising a mixture of an antigen and an adjuvant amount of an interleukin selected from the group consisting of interleukin-1α, interleukin-1β, interleukin-2, interleukin-3, interleukin-4, interleukin-5, interleukin-6, interleukin-7, or mixtures thereof, adsorbed onto an aqueous suspension of alum (e.g., aluminum hydroxide or aluminum phosphate) and a pharmaceutically acceptable preservative, in a pharmaceutically acceptable vehicle and optional adjuvant.

2. The vaccine composition of Claim 1, wherein the antigen is an antigen selected from the group consisting of bacteria, viruses, macro-components of cells, proteins, peptides, glycoproteins, carbohydrates, parasites, fungi, oncogene products and cancer cells.

3. The vaccine composition of Claim 2, wherein the antigen is a bacterial antigen selected from the group consisting of bacterial capsular polymer, oligomer or fragment thereof, and bacterial surface or outer membrane protein.

4. The vaccine composition of Claim 2, wherein the bacterial antigen is from a bacterial pathogen selected from the group consisting of Haemophilus influenzae, Escherichia coli, Neisseria meningitidis, Streptococcus pneumoniae, Streptococcus pyogenes, Branhamella catarrhalis, Vibrio cholerae, Corynebacteria diphtheriae, Neisseria gonorrhoeae, Bordetella pertussis, Pseudomonas aeruginosa, Staphylococcus aureus, Klebsiella pneumoniae and Clostridium tetani.

5. The vaccine composition of Claim 3, wherein the bacterial capsular polymer, oligomer or fragment thereof is Haemophilus influenzae, Streptococcus pneumoniae, Neisseria meningitidis, Klebsiella pneumoniae, Pseudomonas aeruginosa or Staphylococcus aureus.

6. The vaccine composition of Claim 1, wherein the antigen is coupled to a glycoconjugate selected from a bacterial toxin of diphtheria, tetanus, pertussis or cross reacting material (CRM), or toxoid thereof.

7. The vaccine composition of Claim 6, wherein the glycoconjugate comprises polyribosylribitolphosphate and CRM₁₉₇ of diphtheria toxin.

8. The vaccine composition of Claim 3, wherein the bacterial surface or outer membrane protein is of Haemophilus influenzae, Neisseria meningitidis, Neisseria gonorrhoeae, or Branhamella cartarrhalis.

9. A vaccine composition of Claim 3, wherein the bacterial surface protein is the M protein from Streptococcus pyogenes.

10. A vaccine composition of Claim 2, wherein the viral antigen is selected from the group consisting of F protein of respiratory syncytial virus, N protein of respiratory syncytial virus, G protein of respiratory syncytial virus, VP4 polypeptide of rotavirus, VP6 polypeptide of rotavirus, VP7 polypeptide of rotavirus, envelope glycoproteins of human immunodeficiency virus, herpes glycoproteins B and D and the S and pre-S antigens of hepatitis B.

11. A stable vaccine composition comprising, a mixture of an antigen and an adjuvant amount of interleukin-1α, interleukin-1β, interleukin-3, interleukin-4, interleukin-5, or interleukin-7, or mixtures thereof, adsorbed onto an aqueous suspension of alum (e.g., aluminum hydroxide or aluminum phosphate), in a pharmaceutically acceptable vehicle and optional adjuvant.

12. A method of stabilizing an interleukin in a vaccine composition comprising admixing an antigen and an adjuvant amount of interleukin adsorbed onto an aqueous suspension of alum (e.g. aluminum hydroxide or aluminum phosphate).

13. The method of Claim 12, wherein the interleukin is selected from the group consisting of interleukin-1α, interleukin-1β, interleukin-2, interleukin-3, interleukin-4, interleukin-5, interleukin-6, interleukin-7, or mixtures thereof.

## Patentansprüche

1. Stabile Impfstoff-Zusammensetzung, umfassend eine Mischung eines Antigens und einer Adjuvans-Menge eines Interleukins, das aus der Gruppe ausgewählt ist, die aus Interleukin-1α, Interleukin-1β, Interleukin-2, Interleukin-3, Interleukin-4, Interleukin-5, Interleukin-6, Interleukin-7 und Mischungen derselben besteht, adsorbiert auf einer wäßrigen Suspension von Alaun (z.B. Aluminiumhydroxid oder Aluminiumphosphat), und ein pharmazeutisch annehmbares Konservierungsmittel in einem pharmazeutisch annehmbaren Vehikel und fakultativen Adjuvans.

2. Impfstoff-Zusammensetzung nach Anspruch 1, in der das Antigen ein Antigen ist, das aus der Gruppe ausgewählt ist, die aus Bakterien, Viren, Makro-Komponenten von Zellen, Proteinen, Peptiden, Glykoproteinen, Kohlehydraten, Parasiten, Pilzen, Onkogen-Produkten und Krebszellen besteht.

3. Impfstoff-Zusammensetzung nach Anspruch 2, in der das Antigen ein bakterielles Antigen ist, das aus der Gruppe ausgewählt ist, die aus Bakterien-Kapsel-Polymer, -Oligomer oder Fragment derselben und bakteriellem Oberflächen- oder Außenmembran-Protein besteht.

4. Impfstoff-Zusammensetzung nach Anspruch 2, in der das bakterielle Antigen aus einem bakteriellen Pathogen stammt, das aus der Gruppe ausgewählt ist, die aus Haemophilus influenzae, Escherichia coli, Neisseria meningitidis, Streptococcus pneumoniae, Streptococcus pyogenes, Branhamella catarrhalis, Vibrio cholerae, Corynebacteria diphtheriae, Neisseria gonorrhoeae, Bordetella pertussis, Pseudomonas aeruginosa, Staphylococcus aureus, Klebsiella pneumoniae und Clostridium tetani besteht.

5. Impfstoff-Zusammensetzung nach Anspruch 3, in der das Bakterien-Kapsel-Polymer, -Oligomer oder Fragment desselben Haemophilus influenzae, Streptococcus pneumoniae, Neisseria meningitidis, Klebsiella pneumoniae, Pseudomonas aeruginosa oder Staphylococcus aureus ist.

6. Impfstoff-Zusammensetzung nach Anspruch 1, in der das Antigen an ein Glykokonjugat gekuppelt ist, das aus Bakterien-Toxin von Diphtheria, Tetanus, Pertussis oder Kreuz-reagierendem Material (CRM) oder Toxoid derselben ausgewählt ist.

7. Impfstoff-Zusammensetzung nach Anspruch 6, in der das Glykokonjugat Polyribosylribitphosphat und CRM₁₉₇ von Diphtheria-Toxin umfaßt.

8. Impfstoff-Zusammensetzung nach Anspruch 3, in der das bakterielle Oberflächen- oder Außenmembran-Protein von Haemophilus influenzae, Neisseria meningitidis, Neisseria gonorrhoeae oder Branhamella cartarrhalis ist.

9. Impfstoff-Zusammensetzung nach Anspruch 3, in der das bakterielle Oberflächen-Protein das M-Protein von Streptococcus pyogenes ist.

10. Impfstoff-Zusammensetzung nach Anspruch 2, in der das Virus-Antigen ausgewählt ist aus der Gruppe, die aus dem F-Protein des Respiratory Syncytial Virus, dem N-Protein des Respiratory Syncytial Virus, dem G-Protein des Respiratory Syncytial Virus, dem VP4-Polypeptid des Rotavirus, dem VP6-Polypeptid des Rotavirus, dem VP7-Polypeptid des Rotavirus, den Hüllglykoproteinen des Human Immunodeficiency Virus, den Herpes-Glykoproteinen B und D und den S- und Prä-S-Antigenen von Hepatitis B besteht.

11. Stabile Impfstoff-Zusammensetzung, umfassend eine Mischung eines Antigens und einer Adjuvans-Menge Interleukin-1α, Interleukin-1β, Interleukin-3, Interleukin-4, Interleukin-5 oder Interleukin-7 oder Mischungen derselben, adsorbiert auf einer wäßrigen Suspension von Alaun (z.B. Aluminiumhydroxid oder Aluminiumphosphat) in einem pharmazeutisch annehmbaren Vehikel und fakultativen Adjuvans.

12. Verfahren zur Stabilisierung eines Interleukins in einer Impfstoff-Zusammensetzung, umfassend das Mischen eines Antigens und einer Adjuvans-Menge von Interleukin, adsorbiert auf einer wäßrigen Suspension von Alaun (z.B. Aluminiumhydroxid oder Aluminiumphosphat).

13. Verfahren nach Anspruch 12, in dem das Interleukin aus der Gruppe ausgewählt ist, die aus Interleukin-1α, Interleukin-1β, Interleukin-2, Interleukin-3, Interleukin-4, Interleukin-5, Interleukin-6, Interleukin-7 oder Mischungen derselben besteht.

## Revendications

1. Composition vaccinale stable, comprenant un mélange d'un antigène et d'une quantité adjuvante d'une interleukine sélectionnée dans le groupe constitué par l'interleukine-1α, l'interleukine-1β, l'interleukine-2, l'interleukine-3, l'interleukine-4, l'interleukine-5, l'interleukine-6, l'interleukine-7 ou leurs mélanges, adsorée sur une suspension aqueuse d'alun (par exemple hydroxyde d'aluminium ou phosphate d'aluminium) et un agent de conservation pharmaceutiquement acceptable, dans un véhicule pharmaceutiquement acceptable et un adjuvant facultatif.

2. Composition vaccinale selon la revendication 1, dans laquelle l'antigène est un antigène sélectionné dans le groupe constitué par des bactéries, des virus, des macrocomposants cellulaires, des protéines, des peptides, des glycoprotéines, des glucides, des parasites, des champignons, des produits oncogènes et des cellules cancéreuses.

3. Composition vaccinale selon la revendication 2, dans laquelle l'antigène est un antigène bactérien sélectionné dans le groupe constitué par un polymère, ou un oligomère capsulaires bactériens ou un fragment de ces derniers et une protéine de surface ou de membrane externe bactérienne.

4. Composition vaccinale selon la revendication 2, dans laquelle l'antigène bactérien provient d'un agent pathogène bactérien sélectionné dans le groupe constitué par Haemophilus influenzae, Escherichia coli, Neisseria meningitidis, Streptococcus pneumoniae, Streptococcus pyogenes, Branhamella catarrhalis, Vibrio cholerae, Corynebacteria diphtheriae, Neisseria gonorrhoeae, Bordetella pertussis, Pseudomonas aeruginosa, Staphylococcus aureus, Klebsiella pneumoniae et Clostridium tetani.

5. Composition vaccinale selon la revendication 3, dans laquelle le polymère, ou l'oligomère capsulaires bactériens ou leur fragment est Haemophilus influenzae, Streptococcus pneumoniae, Neisseria meningitidis, Klebsiella pneumoniae, Pseudomonas aeruginosa ou Staphylococcus aureus.

6. Composition vaccinale selon la revendication 1, dans laquelle l'antigène est couplé à un glycoconjugué sélectionné parmi une toxine bactérienne de la diphtérie, du tétanos, de la coqueluche ou une substance à réaction croisée (CRM) ou leur anatoxine.

7. Composition vaccinale selon la revendication 6, dans laquelle le glycoconjugué comprend du poly(phosphate de ribosylribitol) et CRM₁₉₇ de la toxine de la diphtérie.

8. Composition vaccinale selon la revendication 3, dans laquelle la protéine de surface ou de membre externe bactérienne provient de Haemophilus influenzae, Neisseria meningitidis, Neisseria gonorrhoeae ou Branhamella catarrhalis.

9. Composition vaccinale selon la revendication 3, dans laquelle la protéine de surface bactérienne est la protéine M de Streptococcus pyogenes.

10. Composition vaccinale selon la revendication 2, dans laquelle l'antigène viral est sélectionné dis le groupe constitué par la protéine F du virus respiratoire syncytial, la protéine N du virus respiratoire syncytial, la protéine G du virus respiratoire syncytial, le polypeptide VP4 du rotavirus, le polypeptide VP6 du rotavirus, le polypeptide VP7 du rotavirus, les glycoprotéines d'enveloppe du virus de l'immunodéficience humaine, les glycoprotéines B et D de l'herpès et les antigènes S et pré-S de l'hépatite B.

11. Composition vaccinale stable, comprenant un mélange d'un antigène et d'une quantité adjuvante d'interleukine-1α, d'interleukine-1β, d'interleukine-3, d'interleukine-4, d'interleukine-5 ou d'interleukine-7 ou leurs mélanges, adsorbés sur une suspension aqueuse d'alun (par exemple hydroxyde d'aluminium ou phosphate d'aluminium) dans un véhicule pharmaceutiquement acceptable et un adjuvant facultatif.

12. Procédé de stabilisation d'une interleukine dans une composition vaccinale, comprenant le mélange d'un antigène et d'une quantité adjuvante d'interleukine adsorbée sur une suspension aqueuse d'alun (par exemple hydroxyde d'aluminium ou phosphate d'aluminium).

13. Procédé selon la revendication 12, dans lequel l'interleukine est sélectionnée dans le groupe constitué par l'interleukine-1α, l'interleukine-1β, l'interleukine-2, l'interleukine-3, l'interleukine-4, l'interleukine-5, l'interleukine-6, l'interleukine-7 ou leurs mélanges.
